# EUROPEAN PATENT APPLICATION

(11) **EP 4 007 274 A1**
(43) Date of publication of application: **01.06.2022**
(21) Application number: 20846540.1
(22) Date of filing: 15.07.2020
(51) Int. Cl.: H04N 7/18, A61B 90/20, G02B 21/36, H04N 5/232

(54) **MEDICAL OBSERVATION SYSTEM, CONTROL DEVICE, AND CONTROL METHOD**

(30) Priority: 29.07.2019 JP 2019138533
(71) Applicant: Sony Group Corporation, Tokyo 108-0075 (JP)
(72) Inventor: KOBAYASHI, Motoaki, Tokyo 108-0075 (JP); KATSUKI, Shinji, Tokyo 108-0075 (JP)
(74) Representative: D Young & Co LLP
(86) International application number: PCT/JP2020/027509
(87) International publication number: WO 2021/020131

(57) **Abstract**

The present disclosure relates to a medical observation system, a control device, and a control method that enable avoidance of losing sight of an observation part.

Generated is an enlarged field-of-view image with a change in field-of-view range from a wide-angle field-of-view image acquired by a capturing device that captures an observation part at a wide angle of view. Then, on the basis of the movement of the capturing device recognized, in a case where it is recognized that the movement of the capturing device has occurred, output is switched from the enlarged field-of-view image to the wide-angle field-of-view image, and thereafter, in a case where it is recognized that the movement of the capturing device has stopped, the output is switched from the wide-angle field-of-view image to the enlarged field-of-view image. The present technology is applicable to, for example, a medical observation system that captures a surgical site as an observation part.

## Description

### TECHNICAL FIELD

The present disclosure relates to a medical observation system, a control device, and a control method, and particularly, to a medical observation system, a control device, and a control method that enable avoidance of losing sight of an observation part.

### BACKGROUND ART

In recent years, medical camera devices that enable enlargement view with a video image have appeared, and such medical camera devices have come to be used for surgery instead of using, for surgery, optical devices such as a microscope and a loupe.

For example, Patent Document 1 discloses a medical observation device capable of switching display between an image having a narrower field-of-view region and an image having a wider field-of-view region in accordance with the detection result indicating whether or not a medical instrument is shown.

### CITATION LIST

### PATENT DOCUMENT

Patent Document 1: Japanese Patent Application Laid-Open No. 2017-38285

### SUMMARY OF THE INVENTION

### PROBLEMS TO BE SOLVED BY THE INVENTION

Meanwhile, enlargement observation with such a medical camera device requires movement of the field of view with movement of the camera in some cases. At this time, in enlargement view of an affected part in a narrower field-of-view region, even if a change in the position or posture of the camera is slight, the affected part under observation may fall outside the screen, resulting in losing sight of the observation part. Thus, required is work of changing again the position or posture of the camera such that the affected part outside the screen falls within the field of view, so that there is a concern that the surgical time will be extended.

For example, in the medical observation device disclosed in Patent Document 1 described above, it is sensed whether or not the medical instrument is shown from the detail of the image being captured and control is performed in accordance with the sensing result. Thus, no control is performed with a specific subject such as the medical instrument not shown in the image. However, in actual surgery, a capturing target differs depending on a surgical method, and thus it is considered not preferable to rely on whether or not control is performed on the basis of only a specific subject.

The present disclosure has been made in view of such a situation, and an object of the present disclosure is to enable avoidance of losing sight of an observation part.

### SOLUTIONS TO PROBLEMS

A medical observation system according to one aspect of the present disclosure includes: a field-of-view range changing unit configured to generate an enlarged field-of-view image with a change in field-of-view range from a wide-angle field-of-view image acquired by a capturing device that captures an observation part at a wide angle of view; a movement recognition unit configured to recognize movement of the capturing device; and an image processing unit configured to make an output with a switch between the wide-angle field-of-view image and the enlarged field-of-view image, on the basis of the movement of the capturing device recognized by the movement recognition unit.

A control device according to one aspect of the present disclosure includes: a field-of-view range changing unit configured to generate an enlarged field-of-view image with a change in field-of-view range from a wide-angle field-of-view image acquired by a capturing device that captures an observation part at a wide angle of view; a movement recognition unit configured to recognize movement of the capturing device; and an image processing unit configured to make an output with a switch between the wide-angle field-of-view image and the enlarged field-of-view image, on the basis of the movement of the capturing device recognized by the movement recognition unit.

A control method for a control device according to one aspect of the present disclosure includes: generating, with a control device, an enlarged field-of-view image with a change in field-of-view range from a wide-angle field-of-view image acquired by a capturing device that captures an observation part at a wide angle of view; recognizing movement of the capturing device; and making an output with a switch between the wide-angle field-of-view image and the enlarged field-of-view image, on the basis of the movement of the capturing device recognized.

In one aspect of the present disclosure, an enlarged field-of-view image with a change in field-of-view range is generated from a wide-angle field-of-view image acquired by a capturing device that captures an observation part at a wide angle of view, movement of the capturing device is recognized, and on the basis of the movement of the capturing device recognized, an output is made with a switch between the wide-angle field-of-view image and the enlarged field-of-view image.

### BRIEF DESCRIPTION OF DRAWINGS

Fig. 1 explanatorily illustrates a situation where a medical observation system is used.
Fig. 2 is a block diagram illustrating an exemplary configuration of an embodiment of the medical observation system to which the present technology is applied.
Fig. 3 is explanatorily illustrates image processing with movement of a capturing device.
Fig. 4 is an explanatory flowchart of exemplary control processing.
Fig. 5 explanatorily illustrates adjustment of an observation position with a center marker.
Fig. 6 is a block diagram illustrating an exemplary configuration of an embodiment of a computer to which the present technology applied.

### MODE FOR CARRYING OUT THE INVENTION

Hereinafter, specific embodiments to which the present technology is applied will be described in detail with reference to the drawings.

### <Exemplary Configuration of Medical Observation System>

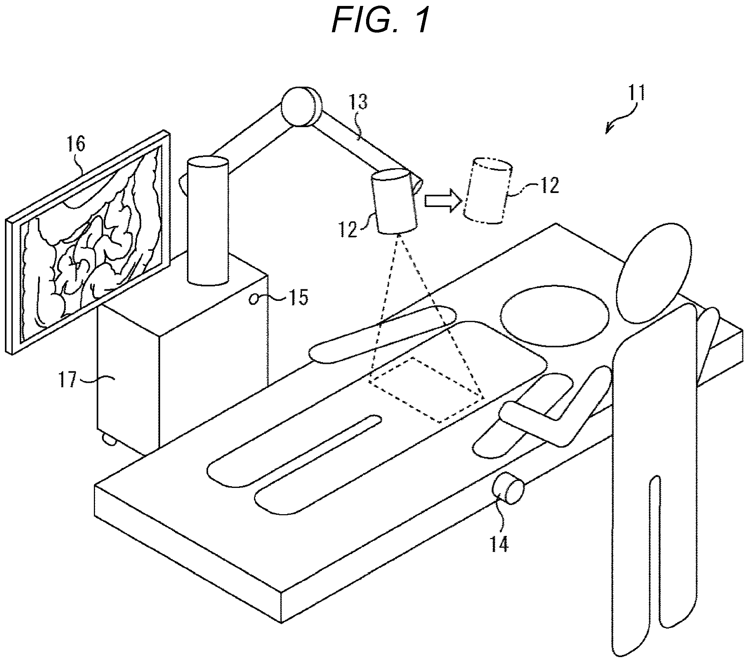

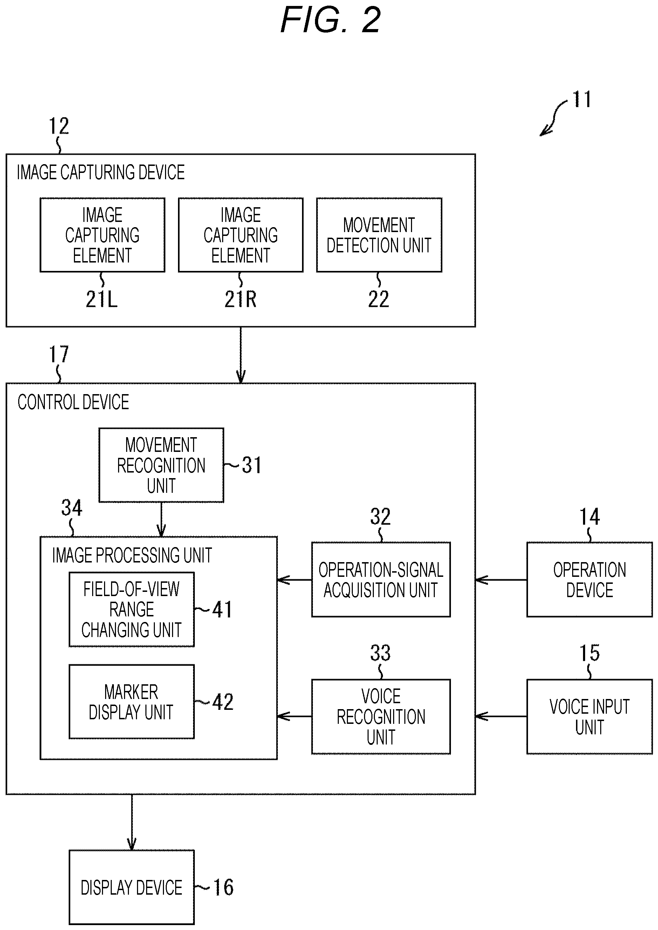

An exemplary configuration of an embodiment of a medical observation system to which the present technology is applied will be described with reference to Figs. 1 and 2. Fig. 1 schematically illustrates a situation where a medical observation system 11 is used, and Fig. 2 is a block diagram illustrating an exemplary configuration of the medical observation system 11.

For example, as illustrated in Fig. 1, the medical observation system 11 is used when surgery is performed on a patient on a patient bed, captures a surgical site as an observation part, and is operated by an operator such as a surgeon or a nurse. In addition, the medical observation system 11 includes a capturing device 12, a support arm 13, an operation device 14, a voice input device 15, a display device 16, and a control device 17.

Furthermore, as illustrated in Fig. 2, the capturing device 12 includes capturing elements 21L and 21R, and a movement detection unit 22. In addition, the control device 17 includes a movement recognition unit 31, an operation-signal acquisition unit 32, a voice recognition unit 33, and an image processing unit 34. The image processing unit 34 includes a field-of-view range changing unit 41 and a marker display unit 42.

The capturing device 12 is set at an angle of field of view such that the entirety of an affected part of the patient can be looked down, and captures, for example, a capturing range surrounded by a broken line in Fig. 1. For example, as the capturing device 12, provided is a stereo camera capable of capturing a stereoscopic image with the capturing element 21L for the left eye and the capturing element 21R for the right eye. Then, the image captured by the capturing elements 21L and 21R is transmitted from the capturing device 12 to the control device 17 through a cable (not illustrated).

Furthermore, in the capturing device 12, the movement detection unit 22 can detect movement of the capturing device 12. For example, when the operator moves the capturing device 12, the movement of the capturing device 12 is detected by the movement detection unit 22, and a movement detection signal indicating the movement of the capturing device 12 is transmitted from the capturing device 12 to the control device 17 through the cable (not illustrated). For example, as the movement detection unit 22, an accelerometer, a gyro sensor, and the like can be provided in combination. The position and posture of the capturing device 12 are specified by the control device 17 on the basis of a movement detection signal indicating acceleration, angular velocity, or the like.

The support arm 13 is a support means having an arm mechanism supporting the capturing device 12, and can support the capturing device 12 at any position by change of the angle of a joint of the arm mechanism. For example, the capturing device 12 is fixed to the distal end of the support arm 13 such that the posture can be changed, and the proximal end of the support arm 13 is rotatably fixed to the control device 17.

Further, the support arm 13 allows the position or the posture of the capturing device 12 to move freely, in response to turning of the joint of the arm mechanism due to an amount of force operable with a hand of the operator only when an external force is applied from the arm mechanism with, for example, gas pressure, spring force, or frictional force. In addition, the support arm 13 maintains the position and posture of the capturing device 12, with no external force applied. Thus, the support arm 13 has a configuration such that the capturing device 12 is moved in accordance with movement of the distal end with the capturing device 12 fixed, while being gripped by the hand of the operator, and such that the capturing device 12 is held at the position and posture when the operator stops the movement of the hand.

The operation device 14 acquires an operation signal responsive to an operation by the operator and supplies the operation signal to the control device 17. For example, on the operation device 14, performed can be a rotating operation of clockwise and counterclockwise rotation, and a tilting operation of tilting in the upward-and-downward direction and the leftward-and-rightward direction. Further, the operation device 14 allows a tilting operation of tilting in a direction in combination of the upward-and-downward direction and the leftward-and-rightward direction, such as a diagonal direction.

For example, a rotating operation on the operation device 14 enables input of an instruction command for an enlargement or a reduction in size of the field-of-view range of an image displayed on the display device 16. In addition, a tilting operation on the operation device 14 enables input of an instruction command for movement of the field-of-view range of the image displayed on the display device 16.

For example, the voice input device 15 includes a microphone, acquires voice uttered by the operator, and supplies a voice signal indicating the voice to the control device 17. For example, similarly to an instruction command input with the operation device 14, the operator can input an instruction command with voice through the voice input device 15. Note that, in addition to being provided as part of the control device 17 as illustrated in Fig. 1, the voice input device 15 may adopt a configuration in which the voice input device 15 is mounted on the head of the operator, provided as part of the display device 16, or incorporated in the capturing device 12 or the support arm 13, for example.

The display device 16 displays an image captured by the capturing device 12, an image obtained by performing image processing on the captured image in the control device 17, and the like. Further, in a case where a stereo image is captured by the display device 16, the display device 16 can stereoscopically display the stereo image.

The control device 17 performs control necessary for image processing on an image captured by the capturing device 12, on the basis of movement of the capturing device 12, an instruction command input with the operation device 14 or the voice input device 15, or the like.

The movement recognition unit 31 acquires the movement detection signal output from the movement detection unit 22, and recognizes the movement of the capturing device 12 in accordance with, for example, acceleration or angular velocity. Then, in a case where the amount of the movement of the capturing device 12 obtained from the movement detection signal is not less than a predetermined threshold, the movement recognition unit 31 notifies the image processing unit 34 that the movement of the capturing device 12 has occurred. At this time, the movement recognition unit 31 notifies the image processing unit 34 of the position and posture of the capturing device 12 obtained from the movement detection signal. Otherwise, in a case where the amount of the movement of the capturing device 12 obtained from the movement detection signal is less than the predetermined threshold, the movement recognition unit 31 notifies the image processing unit 34 that the movement of the capturing device 12 has stopped.

The operation-signal acquisition unit 32 acquires the operation signal output from the operation device 14, and supplies an instruction command in accordance with the acquired operation signal to the image processing unit 34. For example, in response to acquisition of an operation signal indicating a rotating operation on the operation device 14, the operation-signal acquisition unit 32 supplies, to the image processing unit 34, an instruction command for an enlargement or a reduction in size of the field-of-view range in accordance with the rotating direction. In addition, in response to acquisition of an operation signal indicating a tilting operation on the operation device 14, the operation-signal acquisition unit 32 supplies, to the image processing unit 34, an instruction command for movement of the field-of-view range responsive to the tilting direction.

The voice recognition unit 33 acquires the voice signal output from the voice input device 15 and recognizes the detail of the utterance by the operator in accordance with the acquired voice signal. Then, in a case of recognizing an instruction command with voice from the detail of the utterance by the operator, the voice recognition unit 33 supplies the instruction command to the image processing unit 34. For example, in a case of recognizing, with voice, an instruction command of "zoom up" or "zoom down", the voice recognition unit 33 supplies, to the image processing unit 34, an instruction command for an enlargement or a reduction in size of the field-of-view range. Further, in a case of recognizing an instruction command of "move in oo direction", the voice recognition unit 33 supplies, to the image processing unit 34, an instruction command for movement of the field-of-view range in the direction.

On the basis of the movement of the capturing device 12 recognized by the movement recognition unit 31, the image processing unit 34 makes an output, to the display device 16, with a switch between a wide-angle field-of-view image that is an image with a wider field-of-view range and an enlarged field-of-view image that is an image with a narrower field-of-view range resulting from enlargement view of the wide-angle field-of-view image. The wide-angle field-of-view image is an image corresponding to the entire range of the angle of view of the capturing device 12. The enlarged field-of-view image is an image generated by image processing in which the field-of-view range changing unit 41 trims part of the wide-angle field-of-view image and changes the field-of-view range for enlargement view of a desired affected part.

For example, while the enlarged field-of-view image is being output, in a case where the movement recognition unit 31 notifies that the movement of the capturing device 12 has occurred, the image processing unit 34 switches the output to the display device 16, from the enlarged field-of-view image to the wide-angle field-of-view image. At this time, the marker display unit 42 performs image processing of superimposing an enlarged field-of-view marker indicating the field-of-view range of the enlarged field-of-view image at that point in time on a corresponding region in the wide-angle field-of-view image output from the image processing unit 34. Thereafter, in a case where the movement recognition unit 31 notifies that the movement of the capturing device 12 has stopped, the image processing unit 34 switches the output to the display device 16 from the wide-angle field-of-view image to the enlarged field-of-view image. At this time, for example, the display device 16 displays the enlarged field-of-view image resulting from enlargement view of the field-of-view range indicated by the enlarged field-of-view marker superimposed on the wide-angle field-of-view image.

The field-of-view range changing unit 41 performs image processing of trimming part of the wide-angle field-of-view image captured by the capturing device 12 and generating an enlarged field-of-view image. Further, in accordance with the instruction command supplied from the operation-signal acquisition unit 32 or the voice recognition unit 33, the field-of-view range changing unit 41 can perform image processing of enlarging or reducing the field-of-view range of the enlarged field-of-view image and image processing of moving the field-of-view range of the enlarged field-of-view image.

While the image processing unit 34 is outputting the enlarged field-of-view image, in a case where the movement recognition unit 31 notifies that the movement of the capturing device 12 has occurred, the marker display unit 42 stores the field-of-view range of the enlarged field-of-view image at that point in time as the enlarged field-of-view range. Then, the marker display unit 42 performs image processing of superimposing an enlarged field-of-view marker indicating the stored enlarged field-of-view range on a region corresponding to the enlarged field-of-view range in the wide-angle field-of-view image.

Further, on the basis of the position and posture of the capturing device 12 notified from the movement recognition unit 31, while following the movement of the capturing device 12, the marker display unit 42 adjusts the display position and display size of the enlarged field-of-view marker to be superimposed on the wide-angle field-of-view image such that the enlarged field-of-view range stored is indicated. That is, even if the operator moves the capturing device 12, the enlarged field-of-view marker is always displayed on the display device 16 such that the enlarged field-of-view range at the point in time of the occurrence of the movement of the capturing device 12 is indicated. Thus, for example, even if the enlarged field-of-view range is deviated from the center of the wide-angle field-of-view image due to the movement of the capturing device 12 by the operator, the enlarged field-of-view marker is always displayed such that the affected part observed in the enlarged field-of-view image immediately before the movement of the capturing device 12 is surrounded.

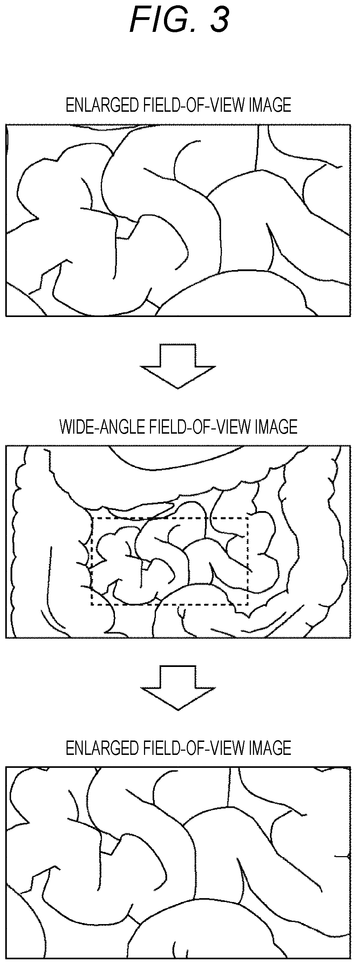

Here, image processing with such movement of the capturing device 12 will be described with reference to Fig. 3.

First, during capturing of an affected part by the capturing device 12 held at the position and posture indicated by a solid line in Fig. 1, such an enlarged field-of-view image as illustrated in the upper part of Fig. 3 is displayed on the display device 16. In the example illustrated in Fig. 3, the enlarged field-of-view image is obtained by trimming of the range of approximately a quarter of the entire range of a wide-angle field-of-view image, for example.

Then, in order to change the observation position, the operator grips the capturing device 12 and starts to move the capturing device 12 toward the position indicated by a two-dot chain line in Fig. 1. At this time, due to detection of the movement of the capturing device 12 by the movement detection unit 22, when the movement recognition unit 31 recognizes that the movement of the capturing device 12 has occurred, the display on the display device 16 is switched so as to display such a wide-angle field-of-view image as illustrated in the middle part of Fig. 3. Here, in the wide-angle field-of-view image of Fig. 3, an enlarged field-of-view marker is represented by a broken-line frame.

Thus, because the wide-angle field-of-view image is displayed on the display device 16 while the operator is moving the capturing device 12, the operator can move the capturing device 12 while viewing the field of view of the entire range that can be captured by the capturing device 12. For example, in the case of the enlarged field-of-view image, even if the position or posture of the capturing device 12 slightly changes, it is highly likely that the affected part falls outside the field of view, resulting in losing sight of the affected part. Thus, in order not to lose sight of such an affected part, the capturing device 12 has required to be moved more finely.

On the other hand, because the wide-angle field-of-view image is set such that the entire surgical site in surgery can be viewed, even if the capturing device 12 is moved quickly, the affected part does not fall outside the field of view immediately after the start of the movement. Thus, the operator can move the capturing device 12 without losing sight of the affected part. In addition, the operator can visually recognize a wider range as the field-of-view range while moving the capturing device 12, resulting in avoidance of a risk that, for example, the capturing device 12 comes into contact with a hand of a person different from the operator, a medical instrument, or the like.

In addition, the wide-angle field-of-view image is displayed with the enlarged field-of-view marker superimposed thereon, so that the operator can move the capturing device 12 while recognizing the observation target in enlargement view at the point in time of the start of the movement of the capturing device 12. Thus, the operator can move the capturing device 12 while recognizing the observation target with reference to the enlarged field-of-view marker, and can predict an enlarged field-of-view image to be displayed when the operator stops the movement of the capturing device 12. Therefore, the operator can move the capturing device 12 such that the enlarged field-of-view image resulting from enlargement view of the desired affected part is displayed on the display device 16 by a single quick operation.

Thereafter, even if the operator stops the movement of the capturing device 12 and releases the hand at the position and posture indicated by the two-dot chain line in Fig. 1, the position and posture of the capturing device 12 are maintained by the arm mechanism of the support arm 13. At this time, due to detection of the movement of the capturing device 12 by the movement detection unit 22, when the movement recognition unit 31 recognizes that the movement of the capturing device 12 has stopped, the display on the display device 16 is switched to display such an enlarged field-of-view image as illustrated in the lower part of Fig. 3.

For example, the field-of-view range changing unit 41 can trim a region corresponding to the enlarged field-of-view range stored in the marker display unit 42 from the wide-angle field-of-view image at the point in time when the movement of the capturing device 12 stops, and can generate the enlarged field-of-view image. Therefore, the operator can bring the affected part in enlargement view before moving the capturing device 12, into similar enlargement view even after moving the capturing device 12.

Furthermore, the medical observation system 11 enables, after a return from the wide-angle field-of-view image to the enlarged field-of-view image, the operator to adjust the observation position and the observation magnification while operating the operation device 14.

Here, an example will be given in which, in the enlarged field-of-view image after the operator stops the movement of the capturing device 12, adjustment is performed for further enlargement observation of an affected part at a position on the left of the affected part displayed on the display device 16 at that time.

First, the operator performs a tilting operation for tilting the operation device 14 leftward, and an operation signal indicating the tilting operation is supplied from the operation device 14 to the control device 17. In response to the supply, in the control device 17, the operation-signal acquisition unit 32 supplies an instruction command for leftward movement of the field-of-view range to the image processing unit 34. Then, while moving, leftward at a predetermined speed, a position where an enlarged field-of-view image is trimmed from the wide-angle field-of-view image, the field-of-view range changing unit 41 generates the enlarged field-of-view image and displays the generated enlarged field-of-view image on the display device 16.

Thereafter, when the operator ends the tilting operation of the operation device 14 at the point in time of display of the affected part to be observed on the display device 16, the operation-signal acquisition unit 32 supplies an instruction command for stop of the movement of the field-of-view range to the image processing unit 34. Accordingly, the field-of-view range changing unit 41 stops the image processing of moving a position where an enlarged field-of-view image is trimmed from the wide-angle field-of-view image, and the enlarged field-of-view image trimmed from the wide-angle field-of-view image at the position at that point in time is continuously displayed on the display device 16.

Subsequently, the operator performs a rotating operation for rotating the operation device 14 clockwise, and an operation signal indicating the rotating operation is supplied from the operation device 14 to the control device 17. In response to the supply, in the control device 17, the operation-signal acquisition unit 32 supplies an instruction command for enlargement of the field-of-view range to the image processing unit 34. Then, while narrowing, at a predetermined speed, a range in which an enlarged field-of-view image is trimmed from the wide-angle field-of-view image, the field-of-view range changing unit 41 generates the enlarged field-of-view image and displays the generated enlarged field-of-view image on the display device 16.

Thereafter, when the operator ends the rotating operation of the operation device 14 at the point in time of display of the affected part to be observed on the display device 16 at a desired magnification, in the control device 17, the operation-signal acquisition unit 32 supplies an instruction command for stop of the enlargement of the field-of-view range to the image processing unit 34. Accordingly, the field-of-view range changing unit 41 stops the image processing of narrowing a range in which an enlarged field-of-view image is trimmed from the wide-angle field-of-view image, and the enlarged field-of-view image trimmed from the wide-angle field-of-view image in the range at that point in time is continuously displayed on the display device 16.

In such a manner, the medical observation system 11 enables the operator to adjust the observation position and the observation magnification such that the desired affected part can be observed at the desired magnification by the operations on the operation device 14. Note that, here, the example of the enlargement of the observation magnification due to the leftward movement of the observation position has been given, but this example is not limiting. Thus, the operator can freely adjust the movement direction of the observation position, the enlargement or reduction of the observation magnification, or the like, and can perform, for example operations on the operation device 14 in any order.

Further, similar to an operation on the operation device 14, in the medical observation system 11, input of an instruction command through the voice input device 15 enables adjustment of the observation position and the observation magnification.

An example will be given in which, in the enlarged field-of-view image after the operator stops the movement of the capturing device 12, adjustment is performed for further enlargement observation of an affected part at a position on the left of the affected part displayed on the display device 16 at that time.

First, when the operator utters "move leftward", the voice is supplied to the control device 17 by the voice input device 15. In response to the supply, in the control device 17, the voice recognition unit 33 recognizes the detail of the utterance, and supplies an instruction command for leftward movement of the field-of-view range to the image processing unit 34. Then, while moving, leftward at a predetermined speed, a position where an enlarged field-of-view image is trimmed from the wide-angle field-of-view image, the field-of-view range changing unit 41 generates the enlarged field-of-view image and displays the generated enlarged field-of-view image on the display device 16.

Thereafter, when the operator utters "stop" at the point in time of display of the affected part to be observed on the display device 16, in the control device 17, the voice recognition unit 33 recognizes the detail of the utterance, and supplies an instruction command for stop of the movement of the field-of-view range to the image processing unit 34. Accordingly, the field-of-view range changing unit 41 stops the image processing of moving a position where an enlarged field-of-view image is trimmed from the wide-angle field-of-view image, and the enlarged field-of-view image trimmed from the wide-angle field-of-view image at the position at that point in time is continuously displayed on the display device 16.

Subsequently, when the operator utters "zoom up", the voice is supplied to the control device 17 by the voice input device 15. In response to the supply, in the control device 17, the voice recognition unit 33 recognizes the detail of the utterance, and supplies an instruction command for enlargement of the field-of-view range to the image processing unit 34. Then, while narrowing, at a predetermined speed, a range in which an enlarged field-of-view image is trimmed from the wide-angle field-of-view image, the field-of-view range changing unit 41 generates the enlarged field-of-view image and displays the generated enlarged field-of-view image on the display device 16.

Thereafter, when the operator utters "stop" at the point in time of display of the affected part to be observed on the display device 16 at a desired magnification, in the control device 17, the voice recognition unit 33 recognizes the detail of the utterance, and supplies an instruction command for stop of the enlargement of the field-of-view range to the image processing unit 34. Accordingly, the field-of-view range changing unit 41 stops the image processing of narrowing a range in which an enlarged field-of-view image is trimmed from the wide-angle field-of-view image, and the enlarged field-of-view image trimmed from the wide-angle field-of-view image in the range at that point in time is continuously displayed on the display device 16.

As described above, in the medical observation system 11, after moving the capturing device 12 to display a desired enlarged field-of-view image on the display device 16, in a case where the operator desires to change the observation position and the observation magnification in the enlarged field-of-view image, the operator can quickly adjust the observation position and the observation magnification. Furthermore, the medical observation system 11 can repeatedly perform processing similar to the above processing, by the movement of the capturing device 12 after the adjustment of the observation position and the observation magnification. That is, processing is repeatedly performed in which a wide-angle field-of-view image is displayed on the display device 16 during the movement of the capturing device 12 and an enlarged field-of-view image resulting from enlargement of the field-of-view range indicated by an enlarged field-of-view marker is displayed on the display device 16 when the movement of the capturing device 12 stops.

Thus, the medical observation system 11 can avoid losing sight of an observation part with the movement of the capturing device 12, so that the operator can quickly repeat the work of moving the capturing device 12 and the work of adjusting the observation position and the observation magnification. This arrangement enables reduction of time loss due to losing sight of the observation part, resulting in avoidance of unnecessary lengthening of the surgical time.

Furthermore, in a case where the configuration in which an instruction command is input with voice through the voice input device 15 is adopted to the medical observation system 11, it is not necessary to dispose the operation device 14 at the position of a hand of the operator, so that the degree of freedom of surgical layout can be increased.

Here, for example, unlike the medical observation device disclosed in Patent Document 1 described above, the medical observation system 11 does not have a configuration in which the medical observation system 11 performs determination based on the detail of an image being captured. Thus, the medical observation system 11 can switch between an enlarged field-of-view image and a wide-angle field-of-view image without depending on a specific subject, resulting in further expansion of the versatility.

In addition, in the case of the medical observation device disclosed in Patent Document 1 described above, the center position of the wide angle view and that of the enlargement view basically agree with each other. The medical observation system 11, however, switches the display from the wide-angle field-of-view image to the enlarged field-of-view image on the basis of the field-of-view range of the immediately preceding enlarged field-of-view image. Thus, the medical observation system 11 enables the operator to predict easily the enlarged field-of-view image after the switching from the wide-angle field-of-view image, so that the operability is further preferable.

Further, the medical observation system 11 enables the adjustment of the observation position and the observation magnification after the switching of the display from the wide-angle field-of-view image to the enlarged field-of-view image, so that the operator can reliably continue to observe an affected part to be subjected to enlargement view.

### <Exemplary Control Processing of Medical Observation System>

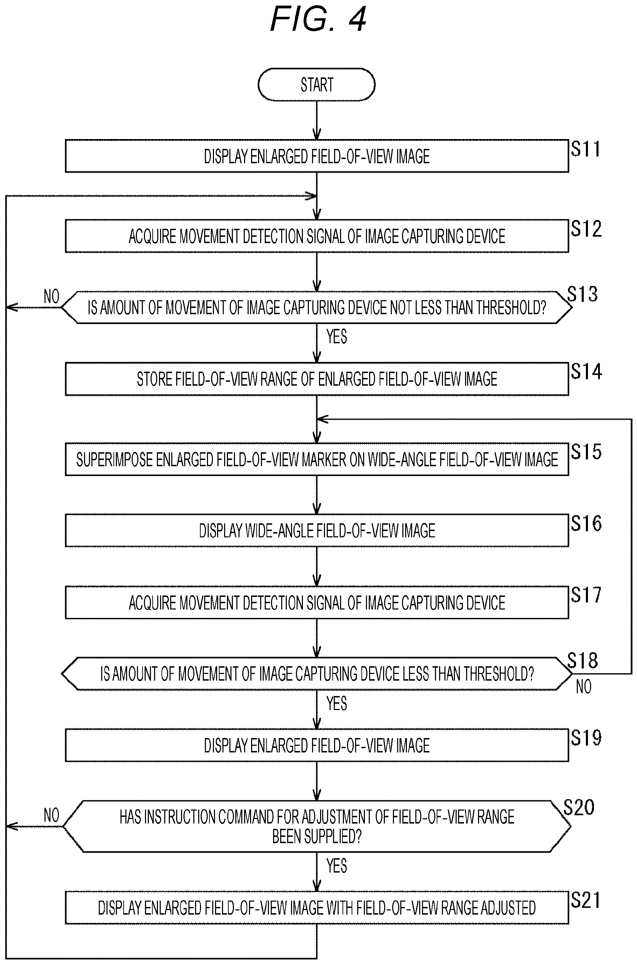

Exemplary control processing executed in the control device 17 will be described with reference to the flowchart illustrated in Fig. 4.

For example, in response to input of an instruction command for display of an enlarged field-of-view image, the processing starts. In step S11, the field-of-view range changing unit 41 performs image processing of trimming part of a wide-angle field-of-view image captured by the capturing device 12 and generating an enlarged field-of-view image. Then, the image processing unit 34 supplies the enlarged field-of-view image generated by the field-of-view range changing unit 41 to the display device 16 to display the enlarged field-of-view image.

In step S12, the movement recognition unit 31 acquires a movement detection signal of the capturing device 12 output from the movement detection unit 22.

In step S13, on the basis of the movement detection signal acquired in step S12, the movement recognition unit 31 determines whether or not the amount of the movement of the capturing device 12 is not less than a predetermined threshold.

In step S13, in a case where the movement recognition unit 31 determines that it is not satisfied that the amount of the movement of the capturing device 12 is not less than the predetermined threshold, namely, in a case where the movement recognition unit 31 determines that the amount of the movement of the capturing device 12 is less than the predetermined threshold, the processing returns to step S12. Namely, in this case, the capturing device 12 is not moved, and thus the processing is held on standby until the operator moves the capturing device 12.

Otherwise, in step S13, in a case where the movement recognition unit 31 determines that the amount of the movement of the capturing device 12 is not less than the predetermined threshold, the movement recognition unit 31 notifies the image processing unit 34 that the movement of the capturing device 12 has occurred, and the processing proceeds to step S14.

In step S14, the marker display unit 42 stores the field-of-view range of the enlarged field-of-view image immediately before the movement of capturing device 12 as an enlarged field-of-view range.

In step S15, in accordance with the enlarged field-of-view range stored in step S14, the marker display unit 42 performs image processing of superimposing, on a corresponding region in the wide-angle field-of-view image, an enlarged field-of-view marker indicating the field-of-view range of the enlarged field-of-view image immediately before the movement of the capturing device 12.

In step S16, the image processing unit 34 outputs the wide-angle field-of-view image with the enlarged field-of-view marker superimposed thereon by the marker display unit 42 in step S15, to the display device 16 to display the wide-angle field-of-view image with the enlarged field-of-view marker superimposed thereon.

In step S17, the movement recognition unit 31 acquires a movement detection signal of the capturing device 12 output from the movement detection unit 22.

In step S18, on the basis of the movement detection signal acquired in step S17, the movement recognition unit 31 determines whether or not the amount of the movement of the capturing device 12 is less than the predetermined threshold.

In step S18, in a case where the movement recognition unit 31 determines that it is not satisfied that the amount of the movement of the capturing device 12 is less than the predetermined threshold, namely, in a case where the movement recognition unit 31 determines that the amount of the movement of the capturing device 12 is not less than the predetermined threshold, the processing returns to step S15. Thus, in this case, the wide-angle field-of-view image with the enlarged field-of-view marker superimposed thereon in accordance with the movement of the capturing device 12 is continuously displayed on the display device 16.

Otherwise, in step S18, in a case where the movement recognition unit 31 determines that the amount of the movement of the capturing device 12 is less than the predetermined threshold, the movement recognition unit 31 notifies the image processing unit 34 that the movement of the capturing device 12 has stopped, and the processing proceeds to step S19.

In step S19, in the image processing unit 34, the field-of-view range changing unit 41 performs image processing of trimming a region corresponding to the enlarged field-of-view range stored in the marker display unit 42 from the wide-angle field-of-view image and generating an enlarged field-of-view image. Then, the image processing unit 34 supplies the enlarged field-of-view image generated by the field-of-view range changing unit 41 to the display device 16 to display the enlarged field-of-view image.

In step S20, the image processing unit 34 determines whether or not an instruction command for adjustment of the field-of-view range has been supplied from the operation-signal acquisition unit 32 or the voice recognition unit 33.

In a case where the image processing unit 34 determines in step S20 that no instruction command for adjustment of the field-of-view range has been supplied, the processing returns to step S12, and then similar processing is repeatedly performed in the subsequent steps.

Otherwise, in a case where the image processing unit 34 determines in step S20 that an instruction command for adjustment of the field-of-view range has been supplied, the processing proceeds to step S21. That is, in this case, an operation signal responsive to an operation of the operation device 14 by the operator is input to the operation-signal acquisition unit 32, or utterance by the operator is input to the voice recognition unit 33 through the voice input device 15 and an instruction command with voice is recognized.

In step S21, in the image processing unit 34, the field-of-view range changing unit 41 performs image processing of generating an enlarged field-of-view image with the field-of-view range adjusted in accordance with the instruction command input by the operator's operation or with the voice. Then, the image processing unit 34 supplies the enlarged field-of-view image generated by the field-of-view range changing unit 41 to the display device 16 to display the enlarged field-of-view image.

Thereafter, the processing returns to step S12, and then similar processing is repeatedly performed in the subsequent steps until the display of the enlarged field-of-view image ends.

As described above, in the medical observation system 11, the field-of-view range that the display device 16 displays can be automatically widened in accordance with the change of the posture and position of the capturing device 12 due to the movement of the capturing device 12 by the operator. Thus, in enlargement view without losing sight of a desired affected part, the operator can move the capturing device 12 to have a desired position and posture. At this time, the operator can change the position and posture of the capturing device 12 while confirming the enlarged field-of-view marker. Thus, the field-of-view range can be quickly changed by a single operation without being deviated from the desired affected part.

Further, in the medical observation system 11, can be displayed is the enlarged field-of-view image corresponding to the enlarged field-of-view range indicated by the enlarged field-of-view marker when the operator stops the movement of the capturing device 12, namely, the enlarged field-of-view range immediately before the movement of the capturing device 12. Thus, for example, as compared with enlargement of the center of a wide-angle field-of-view image, the operator does not need to move consciously or operate carefully the capturing device 12 such that the desired affected part is disposed at the center of the wide-angle field-of-view image, and thus can move the capturing device 12 more easily and quickly.

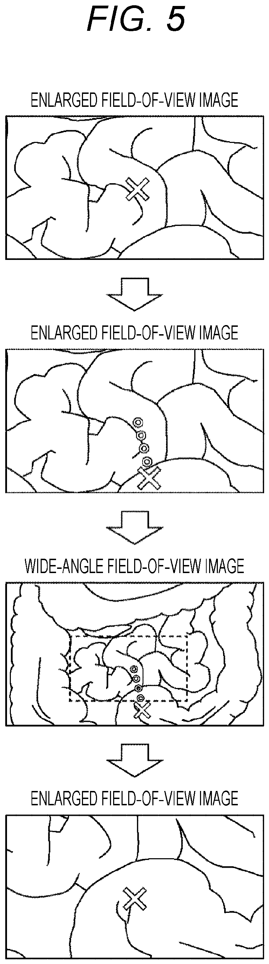

Another exemplary adjustment of the observation position will be described with reference to Fig. 5.

For example, the observation position can be adjusted with such a center marker as indicated by a solid-white cross mark in Fig. 5. For example, when an enlarged field-of-view image is displayed as the movement of the capturing device 12 stops, the center marker is displayed at the center of the enlarged field-of-view image immediately after the movement of the capturing device 12 stops. Then, the operator can move the center marker by operating the operation device 14.

For example, as illustrated in the first part of Fig. 5, the marker display unit 42 displays the center marker at the center of the enlarged field-of-view image immediately after the movement of the capturing device 12 stops.

Then, in response to movement of the center marker due to an operation of the operation device 14 by the operator, the marker display unit 42 moves and displays the center marker in a direction responsive to the operation from the center of the enlarged field-of-view image as illustrated in the second part of Fig. 5. At this time, the marker display unit 42 displays a movement marker that indicates the movement direction of the center marker and linearly connects the center of the enlarged field-of-view image and the center marker. For example, in Fig. 5, such movement markers are each represented by a solid-white double circle.

Further, immediately before the operator moves the center marker to the end of the enlarged field-of-view image while continuing the operation of the operation device 14, the image processing unit 34 switches the display from the enlarged field-of-view image to a wide-angle field-of-view image as illustrated in the third part of Fig. 5. That is, in order to move the position of the enlarged field-of-view image to the wide-angle field-of-view image with the center marker, in a case where the center marker is moved to the end of the enlarged field-of-view image such that the center marker is beyond the field-of-view range of the enlarged field-of-view image being displayed, the wide-angle field-of-view image is displayed such that a range wider than that of the enlarged field-of-view image being displayed can be viewed. At this time, the marker display unit 42 superimposes an enlarged field-of-view marker on the wide-angle field-of-view image similarly to that described above. Thus, the operator can move the center marker while viewing the wide-angle field-of-view image with a wider field-of-view range.

Thereafter, when the movement of the center marker stops in accordance with stop of the operation of the operation device 14 by the operator, the field-of-view range changing unit 41 generates an enlarged field-of-view image with, as the center, the position of the center marker at that point in time. As a result, as illustrated in the fourth part of Fig. 5, the image processing unit 34 switches the display from the wide-angle field-of-view image to the generated enlarged field-of-view image.

As described above, in the medical observation system 11, after the return from the wide-angle field-of-view image to the enlarged field-of-view image, the operator can operate the operation device 14 to adjust the observation position with the center marker.

Note that, in the adjustment of the observation position with the center marker, before the movement of the center marker stops, in accordance with a decrease in the movement speed of the center marker, the field-of-view range may be gradually narrowed from the wide-angle field-of-view image and enlargement display may be performed little by little. In addition, the marker display unit 42 may store in advance the past positions of the center marker as the center history, and each enlarged field-of-view image with the corresponding position as the center may be displayed collectively.

In addition, in the present embodiment, the movement recognition unit 31 determines whether or not the movement of the capturing device 12 has occurred, in accordance with the amount of the movement of the capturing device 12 obtained from the movement detection signal. However, for example, the movement recognition unit 31 may determine whether or not the movement of the capturing device 12 has occurred, on the basis of the absolute value of acceleration or the rate of change in acceleration, the absolute value of angular velocity or the rate of change in angular velocity, or the like detected by the movement detection unit 22.

Further, in accordance with the movement detection signal supplied from the movement detection unit 22, in a case where a change in the movement of the capturing device 12 is less than a certain level, the movement recognition unit 31 does not necessarily notify the image processing unit 34 that the movement of the capturing device 12 has occurred. Otherwise, in accordance with the movement detection signal supplied from the movement detection unit 22, in a case where the change in the movement of the capturing device 12 is not less than the certain level, the movement recognition unit 31 may notify the image processing unit 34 that the movement of the capturing device 12 has occurred. Thus, in this case, even though the amount of the movement of the capturing device 12 is not less than the threshold, if the movement of the capturing device 12 is slower (the change in the movement is less than the certain level), the image processing unit 34 can continue to display the enlarged field-of-view image on the display device 16. That is, when the movement of the capturing device 12 is faster, control is performed such that the wide-angle field-of-view image is displayed on the display device 16 on the assumption that the operator has intentionally moved the capturing device 12.

Note that the present technology is applicable to an endoscope, a video microscope, an operative field camera by open imaging, and the like in addition to such a medical observation system 11 as described above. Furthermore, the present technology may be applied to fields other than medical care.

### <Exemplary Configuration of Computer>

Next, the flow of the processing (control method) described above can be performed by hardware or software. In a case where the flow of the processing is performed by software, a program included in the software is installed, for example, on a general-purpose computer.

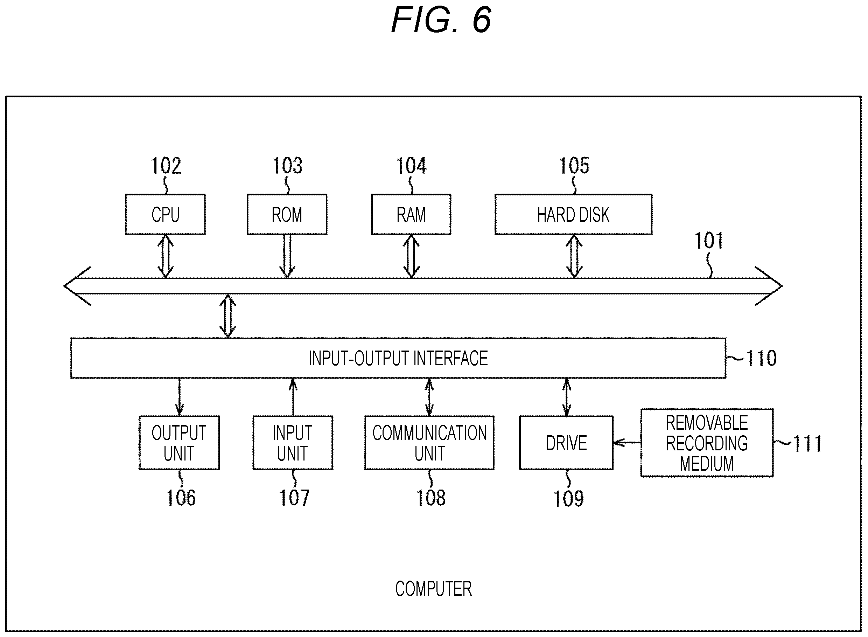

Fig. 6 is a block diagram illustrating an exemplary configuration of an embodiment of a computer on which the program for execution of the flow of the processing described above is installed.

The program can be pre-recorded in a hard disk 105 or a read only memory (ROM) 103 serving as a recording medium built in the computer.

Alternatively, the program can be stored (recorded) in advance in a removable recording medium 111 driven by a drive 109. Such a removable recording medium 111 can be provided as so-called package software. Here, examples of the removable recording medium 111 include a flexible disk, a compact disc read only memory (CD-ROM), a magneto optical (MO) disc, a digital versatile disc (DVD), a magnetic disk, semiconductor memory, and the like.

Note that in addition to installation on the computer from such a removable recording medium 111 as described above, the program can be downloaded to the computer through a communication network or a broadcast network and can be installed in the built-in hard disk 105. Namely, for example, the program can be wirelessly transferred from a download site to the computer through an artificial satellite for digital satellite broadcasting, or can be wiredly transferred to the computer through a network such as a local area network (LAN) or the Internet.

The computer has a built-in central processing unit (CPU) 102, and an input-output interface 110 is connected to the CPU 102 through a bus 101.

When a command is input through the input-output interface 110 due to, for example, an operation of an input unit 107 by the user, the CPU 102 executes the program stored in the ROM 103 in accordance with the command. Alternatively, the CPU 102 loads the program stored in the hard disk 105 into a random access memory (RAM) 104 to execute the program.

As a result, the CPU 102 performs the processing following the above flowchart or the processing performed by the configurations of the above block diagrams. Then, for example, through the input-output interface 110, the CPU 102, as necessary, causes an output unit 106 to output the result of the processing or a communication unit 108 to transmit the result of the processing, and further causes the hard disk 105 to record the result of the processing.

Note that the input unit 107 includes, a keyboard, a mouse, and a microphone. In addition, the output unit 106 includes, a liquid crystal display (LCD) and a speaker.

Here, in the present specification, the processing executed by the computer in accordance with the program is not necessarily performed chronologically in the order described as the flowchart. Namely, the processing executed by the computer in accordance with the program also includes processing executed parallelly or separately (for example, parallel processing or processing with objects).

In addition, the program may be subjected to processing by a single computer (processor) or may be subjected to distributed processing by a plurality of computers. Further, the program may be transferred to a distant computer and may be executed.

Furthermore, in the present specification, the system means a collection of a plurality of constituent elements (devices, modules (components), and others), and thus it is unconcerned whether or not all the constituent elements are provided in the same casing. Therefore, a plurality of devices housed in separate casings and connected through a network, and a single device having a plurality of modules housed in a single casing are both systems.

In addition, for example, the configuration described as a single device (or processing unit) may be divided to provide as a plurality of devices (or a plurality of processing units). Conversely, the configuration described above as a plurality of devices (or a plurality of processing units) may be collectively provided as a single device (or processing unit). In addition, it is needless to say that a configuration different from those described above may be added to the configuration of each device (or each processing unit). Furthermore, if the configuration and operation as the entire system are substantially the same, part of the configuration of a certain device (or processing unit) may be included in the configuration of a different device (or a different processing unit).

In addition, for example, the present technology can adopt a configuration of cloud computing in which a single function is subjected to processing by sharing and collaborating among a plurality of devices through a network.

In addition, for example, the above program can be executed by any device. In that case, it is required that the device has a necessary function (such as a function block) so as to be able to acquire necessary information.

In addition, for example, each step described in the above flowchart can be not only performed by a single device, but also can be performed by sharing among a plurality of devices. Furthermore, in a case where a plurality of pieces of processing is included in a single step, the plurality of pieces of processing included in the single step can be not only performed by a single device, but also can be performed by sharing among a plurality of devices. In other words, a plurality of pieces of processing included in a single step can be performed as a plurality of steps. Conversely, each processing described as a plurality of steps can be collectively performed as a single step.

Note that the program executed by the computer may be provided such that the processing of the steps described in the program is performed chronologically in accordance with the order described in the present specification. Alternatively, the program may be provided such that the processing of the steps described in the program is performed parallelly, or separately with necessary timing, for example, when a call is made. That is, as long as no inconsistency arises, the program may be provided such that the processing of each step is performed in an order different from that described above. Further, the processing of the steps described in the program may be performed parallelly with processing of another program, or may be performed in combination with the processing of the other program.

Note that the plurality of aspects of the present technology described in the present specification can be carried out solely and independently, as long as no inconsistency arises. Needless to say, any aspects of the plurality of aspects of the present technology may be used together and carried out. For example, part or all of the present technology described in any of the embodiments may be combined with part or all of the present technology described in another embodiment. In addition, part or all of any of the plurality of aspects of the present technology described above may be carried out in combination with another technology that is not described above.

### <Exemplary Configuration Combinations>

Note that the present technology can also adopt the configurations below.
(1) A medical observation system including:
   a field-of-view range changing unit configured to generate an enlarged field-of-view image with a change in field-of-view range from a wide-angle field-of-view image acquired by a capturing device that captures an observation part at a wide angle of view;
   a movement recognition unit configured to recognize movement of the capturing device; and
   an image processing unit configured to make an output with a switch between the wide-angle field-of-view image and the enlarged field-of-view image, on the basis of the movement of the capturing device recognized by the movement recognition unit.
(2) The medical observation system according to (1) described above, further including:
   the capturing device; and
   a supporter supporting the capturing device,
   in which the supporter allows, in response to application of an external force to the capturing device, a position or a posture of the capturing device to move freely, and
   the supporter maintains, with no external force applied to the capturing device, a predetermined position and a predetermined posture of the capturing device.
(3) The medical observation system according to (1) or (2) described above,
   in which the capturing device captures a surgical site as the observation part, and
   the field-of-view range changing unit generates the enlarged field-of-view image for enlargement view of a desired affected part in surgery.
(4) The medical observation system according to any of (1) to (3) described above,
   in which the capturing device is provided with a movement detection unit that detects the movement of the capturing device and outputs a movement detection signal indicating the movement of the capturing device, and
   the movement recognition unit obtains an amount of the movement of the capturing device from the movement detection signal, and recognizes that the movement of the capturing device has occurred, in a case where the amount of the movement of the capturing device is not less than a predetermined threshold, or
   recognizes that the movement of the capturing device has stopped, in a case where the amount of the movement is less than the predetermined threshold.
(5) The medical observation system according to (4) described above,
   in which in a case where the movement recognition unit recognizes that the movement of the capturing device has occurred, the image processing unit switches the output from the enlarged field-of-view image to the wide-angle field-of-view image.
(6) The medical observation system according to (4) or (5) described above,
   in which in a case where the movement recognition unit recognizes that the movement of the capturing device has stopped, the image processing unit switches the output from the wide-angle field-of-view image to the enlarged field-of-view image.
(7) The medical observation system according to (4) described above,
   in which in a case where a change in the movement of the capturing device is not less than a predetermined level, the movement recognition unit notifies the image processing unit that the movement of the capturing device has occurred, and
   in response to the notification of the movement of the capturing device having occurred, from the movement recognition unit, the image processing unit switches the output from the enlarged field-of-view image to the wide-angle field-of-view image.
(8) The medical observation system according to any of (1) to (3) described above,
   in which the image processing unit
   switches the output from the enlarged field-of-view image to the wide-angle field-of-view image in a case where the movement recognition unit recognizes that the movement of the capturing device has occurred, and
   switches the output from the wide-angle field-of-view image to the enlarged field-of-view image in a case where the movement recognition unit recognizes that the movement of the capturing device has stopped.
(9) The medical observation system according to (8) described above, further including:
   a marker display unit configured to store, in the switching of the output from the enlarged field-of-view image to the wide-angle field-of-view image by the image processing unit, the field-of-view range of the enlarged field-of-view image as an enlarged field-of-view range and display an enlarged field-of-view marker indicating the enlarged field-of-view range, in superimposition on a region corresponding to the enlarged field-of-view range in the wide-angle field-of-view image.
(10) The medical observation system according to (9) described above,
   in which on the basis of a position and a posture of the capturing device recognized by the movement recognition unit, while following the movement of the capturing device, the marker display unit adjusts a display position and a display size of the enlarged field-of-view marker such that the enlarged field-of-view range is indicated.
(11) The medical observation system according to (9) or (10) described above,
   in which in the switching of the output from the wide-angle field-of-view image to the enlarged field-of-view image by the image processing unit, the field-of-view range changing unit generates the region corresponding to the enlarged field-of-view range stored in the marker display unit as the enlarged field-of-view image.
(12) The medical observation system according to any of (1) to (11) described above, further including:
   an acquisition unit configured to acquire an instruction command for movement of a position of the enlarged field-of-view image to the wide-angle field-of-view image or for an enlargement or a reduction in magnification of the enlarged field-of-view image to the wide-angle field-of-view image, to supply the instruction command to the field-of-view range changing unit,
   in which the field-of-view range changing unit generates the enlarged field-of-view image from the wide-angle field-of-view image in accordance with the instruction command.
(13) The medical observation system according to (12) described above,
   in which the acquisition unit acquires the instruction command on the basis of an operation signal output from an operation unit on which a rotating operation or a tilting operation is performed by an operator.
(14) The medical observation system according to (12) or (13) described above,
   in which the acquisition unit serves as a voice recognition unit that recognizes a detail of utterance from voice uttered by the operator, to acquire the instruction command.
(15) The medical observation system according to any of (12) to (14) described above,
   in which in the movement of the position of the enlarged field-of-view image to the wide-angle field-of-view image with the enlarged field-of-view image having a center designated, in a case where a range different from a range of the enlarged field-of-view image being displayed is designated on the basis of the center, a wider range than the range of the enlarged field-of-view image being displayed is displayed.
(16) The medical observation system according to (15) described above,
   in which as the display of the wider range than the range of the enlarged field-of-view image being displayed, the display is switched to the wide-angle field-of-view image.
(17) A control device including:
   a field-of-view range changing unit configured to generate an enlarged field-of-view image with a change in field-of-view range from a wide-angle field-of-view image acquired by a capturing device that captures an observation part at a wide angle of view;
   a movement recognition unit configured to recognize movement of the capturing device; and
   an image processing unit configured to make an output with a switch between the wide-angle field-of-view image and the enlarged field-of-view image, on the basis of the movement of the capturing device recognized by the movement recognition unit.
(18) A control method for a control device including:
   generating, with the control device, an enlarged field-of-view image with a change in field-of-view range from a wide-angle field-of-view image acquired by a capturing device that captures an observation part at a wide angle of view;
   recognizing movement of the capturing device; and
   making an output with a switch between the wide-angle field-of-view image and the enlarged field-of-view image, on the basis of the movement of the capturing device recognized.

Note that the embodiments of the present technology are not limited to the above embodiments, and thus various modifications can be made within the scope without departing from the gist of the present disclosure. In addition, the effects described in the present specification are merely exemplified and are not intended to be limiting; thus, there may have additional effects.

### REFERENCE SIGNS LIST

- 11: Medical observation system
- 12: Capturing device
- 13: Support arm
- 14: Operation device
- 15: Voice input device
- 16: Display device
- 17: Control device
- 21L and 21R: Capturing elements
- 22: Movement detection unit
- 31: Movement recognition unit
- 32: Operation-signal acquisition unit
- 33: Voice recognition unit
- 34: Image processing unit
- 41: Field-of-view range changing unit
- 42: Marker display unit

## Claims

1. A medical observation system comprising:
a field-of-view range changing unit configured to generate an enlarged field-of-view image with a change in field-of-view range from a wide-angle field-of-view image acquired by a capturing device that captures an observation part at a wide angle of view;
a movement recognition unit configured to recognize movement of the capturing device; and
an image processing unit configured to make an output with a switch between the wide-angle field-of-view image and the enlarged field-of-view image, on a basis of the movement of the capturing device recognized by the movement recognition unit.

2. The medical observation system according to claim 1, further comprising:
the capturing device; and
a supporter supporting the capturing device,
wherein the supporter allows, in response to application of an external force to the capturing device, a position or a posture of the capturing device to move freely, and
the supporter maintains, with no external force applied to the capturing device, a predetermined position and a predetermined posture of the capturing device.

3. The medical observation system according to claim 1,
wherein the capturing device captures a surgical site as the observation part, and
the field-of-view range changing unit generates the enlarged field-of-view image for enlargement view of a desired affected part in surgery.

4. The medical observation system according to claim 1,
wherein the capturing device is provided with a movement detection unit that detects the movement of the capturing device and outputs a movement detection signal indicating the movement of the capturing device, and
the movement recognition unit obtains an amount of the movement of the capturing device from the movement detection signal, and recognizes that the movement of the capturing device has occurred, in a case where the amount of the movement of the capturing device is not less than a predetermined threshold, or
recognizes that the movement of the capturing device has stopped, in a case where the amount of the movement is less than the predetermined threshold.

5. The medical observation system according to claim 4,
wherein in a case where the movement recognition unit recognizes that the movement of the capturing device has occurred, the image processing unit switches the output from the enlarged field-of-view image to the wide-angle field-of-view image.

6. The medical observation system according to claim 4,
wherein in a case where the movement recognition unit recognizes that the movement of the capturing device has stopped, the image processing unit switches the output from the wide-angle field-of-view image to the enlarged field-of-view image.

7. The medical observation system according to claim 4,
wherein in a case where a change in the movement of the capturing device is not less than a predetermined level, the movement recognition unit notifies the image processing unit that the movement of the capturing device has occurred, and
in response to the notification of the movement of the capturing device having occurred, from the movement recognition unit, the image processing unit switches the output from the enlarged field-of-view image to the wide-angle field-of-view image.

8. The medical observation system according to claim 1,
wherein the image processing unit
switches the output from the enlarged field-of-view image to the wide-angle field-of-view image in a case where the movement recognition unit recognizes that the movement of the capturing device has occurred, and
switches the output from the wide-angle field-of-view image to the enlarged field-of-view image in a case where the movement recognition unit recognizes that the movement of the capturing device has stopped.

9. The medical observation system according to claim 8, further comprising:
a marker display unit configured to store, in the switching of the output from the enlarged field-of-view image to the wide-angle field-of-view image by the image processing unit, the field-of-view range of the enlarged field-of-view image as an enlarged field-of-view range and display an enlarged field-of-view marker indicating the enlarged field-of-view range, in superimposition on a region corresponding to the enlarged field-of-view range in the wide-angle field-of-view image.

10. The medical observation system according to claim 9,
wherein on a basis of a position and a posture of the capturing device recognized by the movement recognition unit, while following the movement of the capturing device, the marker display unit adjusts a display position and a display size of the enlarged field-of-view marker such that the enlarged field-of-view range is indicated.

11. The medical observation system according to claim 9,
wherein in the switching of the output from the wide-angle field-of-view image to the enlarged field-of-view image by the image processing unit, the field-of-view range changing unit generates the region corresponding to the enlarged field-of-view range stored in the marker display unit as the enlarged field-of-view image.

12. The medical observation system according to claim 1, further comprising:
an acquisition unit configured to acquire an instruction command for movement of a position of the enlarged field-of-view image to the wide-angle field-of-view image or for an enlargement or a reduction in magnification of the enlarged field-of-view image to the wide-angle field-of-view image, to supply the instruction command to the field-of-view range changing unit,
wherein the field-of-view range changing unit generates the enlarged field-of-view image from the wide-angle field-of-view image in accordance with the instruction command.

13. The medical observation system according to claim 12,
wherein the acquisition unit acquires the instruction command on a basis of an operation signal output from an operation unit on which a rotating operation or a tilting operation is performed by an operator.

14. The medical observation system according to claim 12,
wherein the acquisition unit serves as a voice recognition unit that recognizes a detail of utterance from voice uttered by the operator, to acquire the instruction command.

15. The medical observation system according to claim 12,
wherein in the movement of the position of the enlarged field-of-view image to the wide-angle field-of-view image with the enlarged field-of-view image having a center designated, in a case where a range different from a range of the enlarged field-of-view image being displayed is designated on a basis of the center, a wider range than the range of the enlarged field-of-view image being displayed is displayed.

16. The medical observation system according to claim 15,
wherein as the display of the wider range than the range of the enlarged field-of-view image being displayed, the display is switched to the wide-angle field-of-view image.

17. A control device comprising:
a field-of-view range changing unit configured to generate an enlarged field-of-view image with a change in field-of-view range from a wide-angle field-of-view image acquired by a capturing device that captures an observation part at a wide angle of view;
a movement recognition unit configured to recognize movement of the capturing device; and
an image processing unit configured to make an output with a switch between the wide-angle field-of-view image and the enlarged field-of-view image, on a basis of the movement of the capturing device recognized by the movement recognition unit.

18. A control method for a control device comprising:
generating, with the control device, an enlarged field-of-view image with a change in field-of-view range from a wide-angle field-of-view image acquired by a capturing device that captures an observation part at a wide angle of view;
recognizing movement of the capturing device; and
making an output with a switch between the wide-angle field-of-view image and the enlarged field-of-view image, on a basis of the movement of the capturing device recognized.
